# EUROPÄISCHE PATENTSCHRIFT

(11) **EP 0 295 501 B1**
(45) Veröffentlichungstag und Bekanntmachung des Hinweises auf die Patenterteilung: **14.04.1993**
(21) Anmeldenummer: 88108806.6
(22) Anmeldetag: 01.06.1988
(51) Int. Cl.: A61B 6/06

(54) **Röntgenzielgerät**
X-ray target apparatus
Appareil avec diaphragme d'objectif radiographique

(30) Priorität: 15.06.1987 DE 8708397 U
(43) Veröffentlichungstag der Anmeldung: 21.12.1988
(73) Patentinhaber: SIEMENS AKTIENGESELLSCHAFT, 80333 München (DE)
(72) Erfinder: Hubert, Günter, D-8523 Baiersdorf (DE); Finkenzeller, Johann, D-8520 Erlangen (DE)

(56) Entgegenhaltungen:
- EP-A- 0 089 094
- DE-A- 2 842 659
- DE-A- 3 034 915
- DE-A- 3 331 555
- DE-A- 3 610 080

## Beschreibung

Die Erfindung betrifft ein Röntgenzielgerät mit einer Sekundärstrahlenblende, einem wahlweise verwendbaren Streustrahlenraster und einem Kassettenschacht, in den eine Röntgenfilmkassette einführbar ist, wobei die Sekundärstrahlenblende zwei Blendenplatten aufweist, die in zueinander parallelen Ebenen angeordnet und mittels einer Stelleinrichtung gegeneinander und übereinander hinweg verstellbar sind.

Ein derartiges Röntgenzielgerät ist in der DE-OS 28 42 659 beschrieben. Dabei sind zwischen jener Wand des Gehäuses des Röntgenzielgerätes, an der das Untersuchungsobjekt anliegt, und dem Schacht für die Röntgenfilmkassette in aufeinanderfolgenden Ebenen die Blendenplatten der Sekundärstrahlenblende und das Streustrahlenraster angeordnet, so daß zwischen einem innerhalb des Untersuchungsobjektes befindlichen abzubildenden Objekt und der Filmebene ein relativ großer Abstand vorliegt. Dies wirkt sich nachteilig auf die Qualität einer Röntgenaufnahme des abzubildenden Objektes aus, deren die Unschärfe bekanntermaßen mit dem Abstand des abzubildenden Objektes von der Filmebene zunimmt.

Der Erfindung liegt deshalb die Aufgabe zugrunde, ein Röntgenzielgerät der eingangs genannten Art so auszubilden, daß im Interesse einer geringen Unschärfe der Abstand eines aufzunehmenden Objektes von der Filmebene möglichst gering ist.

Erfindungsgemäß wird diese Aufgabe dadurch gelöst, daß die Blendenplatten jeweils eine Blendenöffnung besitzen, wobei die Blendenöffnung jeweils einer Blendenplatte durch die jeweils andere Blendenplatte zumindest teilweise abdeckbar ist, und daß die Blendenöffnung einer der Blendenplatten mit dem Streustrahlenraster versehen ist. Da das Streustrahlenraster in eine der ohnehin vorhandenen Blendenplatten integriert ist und die Blendenplatten mittels der Stelleinrichtung so eingestellt werden können, daß sich wahlweise entweder die ohne Streustrahlenraster ausgeführte oder die mit dem Streustrahlenraster versehene Blendenöffnung vor der Röntgenfilmkassette befindet, nimmt das Streustrahlenraster anders als beim Stand der Technik keinen zusätzlichen Bauraum in Richtung des Strahlenganges ein. Damit weist ein aufzunehmendes Objekt im Falle des erfindungsgemäßen Röntgenzielgerätes einen geringeren Abstand von der Filmebene auf als beim Stand der Technik. Da im Falle des erfindungsgemäßen Röntgenzielgerätes die Möglichkeit besteht, die jeweils vor der Röntgenfilmkassette befindliche Blendenöffnung durch Verstellen der anderen Blendenplatte zumindest teilweise abzudecken, kann die Größe der Blendenöffnung an das Format der jeweils anzufertigenden Röntgenaufnahme angepaßt werden.

Eine Ausführungsform der Erfindung sieht vor, daß die Stelleinrichtung mit den Blendenplatten in einer Ebene angeordnet ist. Durch diese Maßnahme ist sichergestellt, daß die Stelleinrichtung keinen negativen Einfluß auf den erzielbaren Abstand zwischen aufzunehmendem Objekt und Filmebene ausübt.

Ein besonders geringer Abstand zwischen dem aufzunehmenden Objekt und der Filmebene ist erzielbar, wenn nach einer Variante der Erfindung die Blendenplatten mit ihren einander zugewandten Flächen unmittelbar aufeinander gleiten. Dabei kann vorgesehen sein, daß eine der Blendenplatten an ihrer der anderen Blendenplatte zugewandten Fläche einen reibungsarmen Belag aufweist, um die Betätigungskräfte möglichst gering zu halten. Um eine ausreichende Führung der Blendenplatten zu gewährleisten, können zu beiden Seiten der Blendenplatten in deren Bewegungsrichtung verlaufende U-förmige Führungsschienen vorgesehen sein, die jeweils beide Blendenplatten mit ihren parallelen Schenkeln umgreifen und mit ihrem anderen Schenkel quer zu deren Bewegungsrichtung führen. In Fällen, in denen es nicht ratsam erscheint, die Blendenplatten unmittelbar aufeinander gleiten zu lassen, kann vorgesehen sein, daß zu beiden Seiten der Blendenplatten in deren Bewegungsrichtung verlaufende T-förmige Führungsschienen vorgesehen sind, wobei die Führungsschienen mit ihrem Steg jeweils zwischen die Blendenplatten greifen und mit ihren Flanschen die Blendenplatten quer zu deren Bewegungsrichtung führen. Eine Verkantung der Blendenplatten in den Führungsschienen kann auf besonders einfache Weise vermieden werden, wenn die Blendenplatten in ihrer Bewegungsrichtung eine Erstreckung aufweisen, die wenigstens ihrer Abmessung quer dazu entspricht.

Eine Variante der Erfindung sieht vor, daß die Stelleinrichtung für die Blendenplatten zwei Zugorgane umfaßt, deren jeweilige Enden an verschiedenen Blendenplatten befestigt sind und die derart um Umlenkrollen geführt sind, daß ihre jeweiligen an den Blendenplatten angebrachten Abschnitte parallel zueinander und die an jeweils einer Blendenplatte angebrachten Abschnitte der Zugorgane entgegengesetzt zueinander verlaufen, wobei eines der Zugorgane antreibbar ist. Durch eine derartige Ausbildung der Stelleinrichtung, die eine synchrone Verstellung der Blendenplatten erlaubt, ist es ohne weiteres möglich, diese mit den Blendenplatten in einer gemeinsamen Ebene anzuordnen, so daß eine durch die Stelleinrichtung bedingte Vergrößerung des Abstandes zwischen dem aufzunehmenden Objekt und der Filmebene nicht auftritt.

Nach einer Ausführungsform der Erfindung ist vorgesehen, daß das Streustrahlenraster streifenförmige Lamellen aufweist, die parallel zur Bewegungsrichtung der Blendenplatten verlaufen. Durch diese Maßnahme ist unter der Voraussetzung, daß das Streustrahlenraster einen festen Abstand zu einer mit dem Röntgenzielgerät zusammenwirkenden Röntgenröhre aufweist, sichergestellt, daß ein fest fokussiertes Streustrahlenraster verwendet werden kann, was im Falle von z.B. quer zur Bewegungsrichtung der Blendenplatten verlaufenden Lamellen nicht möglich wäre.

Eine weitere Variante der Erfindung sieht vor, daß zwischen der Sekundärstrahlenblende und dem Kassettenschacht eine aus einem reibungsarmen Werkstoff gebildete Zwischenwand zur Führung der Röntgenfilmkassette vorgesehen ist.

Ein Ausführungsbeispiel der Erfindung ist in den beigefügten Zeichnungen dargestellt. Es zeigen:
- Fig. 1: einen teilweisen Querschnitt durch ein erfindungsgemäßes Röntgenzielgerät,
- Fig. 2: in schematischer Darstellung einen Schnitt durch das Röntgenzielgerät nach Fig. 1,
- Fig. 3 und 4: in schematischer Darstellung unterschiedliche Betriebszustände des erfindungsgemäßen Röntgenzielgerätes, und
- Fig. 5: eine Einzelheit eines erfindungsgemäßen Röntgenzielgerätes im Querschnitt.

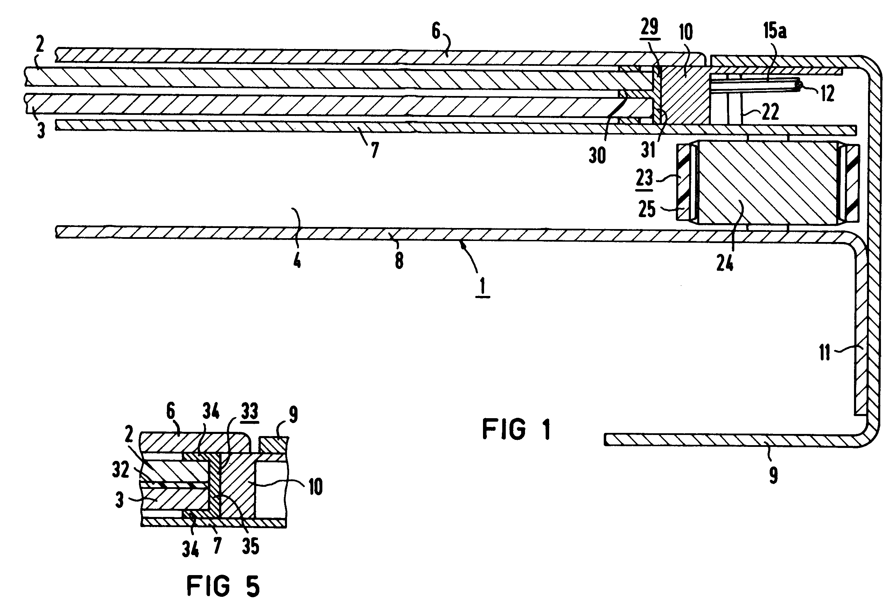

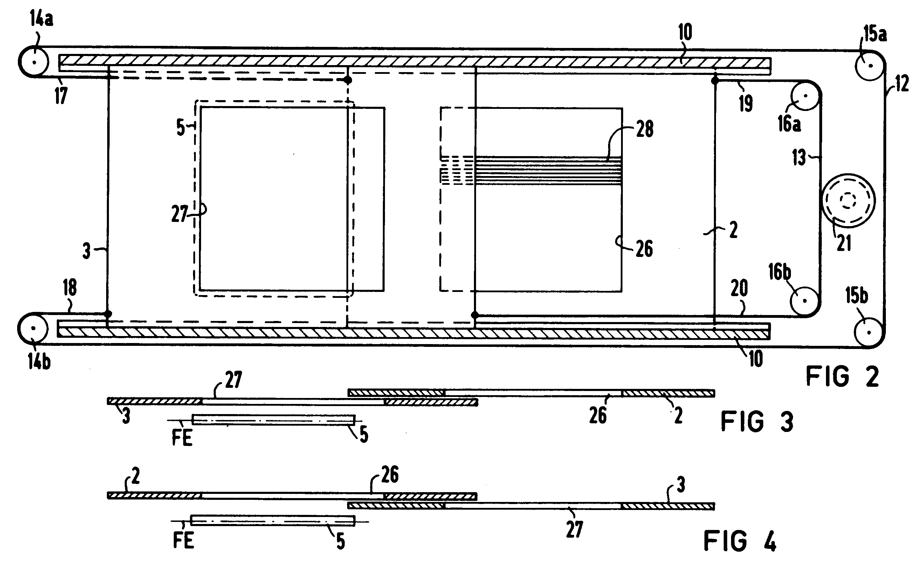

In den Fig. 1 und 2 ist ein erfindungsgemäßes Röntgenzielgerät dargestellt, das wie aus Fig. 1 ersichtlich ein insgesamt mit der Bezugsziffer 1 versehenes Gehäuse aufweist, in dem eine Sekundärstrahlenblende mit zwei aus einem für Röntgenstrahlung undurchlässigen Werkstoff bestehenden Blendenplatten 2 und 3 und ein Kassettenschacht 4, in dem eine in Fig. 1 nicht dargestellte und in Fig. 2 strichliert angedeutete Röntgenfilmkassette 5 einführbar ist, vorgesehen sind. Das Gehäuse 1 weist eine Rückwand 6, an der während der Anfertigung einer Röntgenaufnahme ein Untersuchungsobjekt anliegt, eine Zwischenwand 7 und eine Vorderwand 8 auf, wobei wenigstens die Rückwand 6 und die Zwischenwand 7 aus einem für Röntgenstrahlen durchlässigen Material bestehen. Die Blendenplatten 2 und 3 befinden sich zwischen der Rückwand 6 und der Zwischenwand 7, während letztere gemeinsam mit der Vorderwand 8 den Kassettenschacht 4 begrenzt. Das Gehäuse 1 weist außerdem einen Rahmen 9 auf, mittels dessen das Röntgenzielgerät an einem Röntgenuntersuchungsgerät angebracht werden kann. An dem Rahmen 9 sind zwei Führungsteile 10 für die Blendenplatten 2 und 3 angebracht, wobei die Führungsteile 10 zugleich die Rückwand 6 und die Zwischenwand 7 tragen. Die Vorderwand 8 ist über Abwinkelungen 11 an dem Rahmen 10 befestigt.

Wie aus der Fig. 1 in Verbindung mit der Fig. 2, in der aus Gründen der Übersichtlichkeit das Gehäuse 1 fortgelassen ist, ersichtlich ist, sind die Blendenplatten 2 und 3 in zueinander parallelen Ebenen angeordnet und mit einer Stelleinrichtung versehen. Die Stelleinrichtung umfaßt zwei Zugorgane, nämlich die Seile 12 und 13, deren Enden jeweils an verschiedenen Blendenplatten 2, 3 befestigt sind, d.h. die Seile 12 und 13 sind jeweils mit ihrem einen Ende an der Blendenplatte 2 und mit ihrem anderen Ende an der Blendenplatte 3 befestigt. Das Seil 12 ist über Umlenkrollen 14a, 14b, 15a und 15b und das Seil 13 über Umlenkrollen 16a und 16b in einer solchen Weise geführt, daß die an den Blendenplatten 2 und 3 angebrachten Abschnitte 17 und 18 bzw. 19 und 20 der Seile 12 und 13 jeweils parallel zueinander verlaufen, wobei im Falle des dargestellten Ausführungsbeispieles sämtliche Abschnitte 17 bis 20 parallel zueinander verlaufen. Außerdem sind die Seile 12 und 13 so über die Umlenkrollen 14a bis 16b geführt, daß die an der Blendenplatte 3 angebrachten Abschnitte 17 und 19 und die an der Blendenplatte 2 angebrachten Abschnitte 18 und 20 der Seile 12 und 13 jeweils entgegengesetzt zueinander verlaufen. Das Seil 13 ist um eine motorisch antreibbare Seilrolle 21 geschlungen. Die Blendenplatten 2 und 3 sind demzufolge durch Verdrehen der Seilrolle 21 in der einen oder anderen Richtung synchron gegeneinander und übereinander hinweg verstellbar. Da die Seile 2 und 3 jeweils mit ihrem einen Ende an der Blendenplatte 2 und ihrem anderen Ende an der Blendenplatte 3 befestigt sind und sich die Blendenplatten 2 und 3 in unterschiedlichen Ebenen befinden, sind die Drehachsen der Umlenkrollen 14a bis 16b, wie dies in Fig. 1 anhand der Umlenkrolle 15a und der entsprechenden Drehachse 22 beispielhaft verdeutlicht ist, um ein für einen einwandfreien Lauf der Seile 12 bzw. 13 erforderliches Maß geneigt. Die gesamte Verstelleinrichtung befindet sich übrigens, wie aus Fig. 1 ersichtlich ist, mit den Blendenplatten 2 und 3 in einer gemeinsamen Ebene.

Zum Einführen einer Röntgenfilmkassette 5 in den Kassettenschacht 4 ist eine motorische Transporteinrichtung vorgesehen, die einen Zahnriemen 23 aufweist, der über zwei seitlich neben dem Kassettenschacht 4 angeordnete Riemenscheiben, von denen eine motorisch antreibbar und in Fig. 1 nur die Riemenscheibe 24 sichtbar ist, derart geführt ist, daß sein dem Kassettenschacht zugewandtes Trumm 25 parallel zur Bewegungsrichtung der Röntgenfilmkassette 5 verläuft. Dem Trumm 25 des Zahnriemens 23 gegenüberliegend sind auf der anderen Seite des Kassettenschachtes 4 in Fig. 1 nicht dargestellte Andruckrollen vorgesehen. Sobald eine Röntgenfilmkassette 5 in den Kassettenschacht 4 eingeführt wird, wird z.B. mittels einer durch die Röntgenfilmkassette 5 betätigten Lichtschranke ein Antriebsmotor für den Zahnriemen 23 in Gang gesetzt, so daß die zwischen dem Trumm 25 des Zahnriemens 23 und den Andruckrollen aufgenommene Röntgenfilmkassette in den Kassettenschacht 4 eingezogen wird. Nach erfolgter Röntgenaufnahme wird die Röntgenfilmkassette 5 aus dem Kassettenschacht 4 motorisch heraustransportiert.

Wie aus der Fig. 2 ersichtlich ist, sind die Blendenplatten 2 und 3 jeweils mit einer Blendenöffnung 26 bzw. 27 versehen, wobei die Blendenöffnung 26 der Blendenplatte 2 mit einem Streustrahlenraster 28 versehen ist, dessen Lamellen, von denen in Fig. 2 nur einige eingezeichnet sind, in Bewegungsrichtung der Blendenplatten 2 und 3 verlaufen. Mittels der beschriebenen Stelleinrichtung, die es gestattet, die Blendenplatten 2 und 3 übereinander hinweg zu verstellen, ist es möglich, wie dies die Fig. 3 und 4 in Verbindung mit der Fig. 2 verdeutlichen, wahlweise die Blendenöffnung 26 oder die Blendenöffnung 27 vor der Röntgenfilmkassette 5 zu positionieren, so daß wahlweise Röntgenaufnahmen mit oder ohne Streustrahlenraster angefertigt werden können. Außerdem ist es infolge der synchronen Verstellbarkeit der Blendenplatten 2 und 3 gegeneinander möglich, die jeweils vor der Röntgenfilmkassette 5 befindliche Blendenöffnung 26 oder 27 der Blendenplatte 2 oder 3 mittels der jeweils anderen Blendenplatte 3 oder 2 zumindest teilweise abzudecken, so daß die Größe der Blendenöffnung 26 bzw. 27 dem jeweiligen Aufnahmeformat angepaßt werden kann.

Infolge der beschriebenen Ausbildung des erfindungsgemäßen Röntgenzielgerätes ist der Abstand zwischen der Rückwand 6 des Gehäuses 1 und dem Kassettenschacht 4, d.h. der Filmebene, im Vergleich zum Stand der Technik sehr gering, da eine besondere Ebene für das Streustrahlenraster 28 entfällt und die Blendenplatten 2 und 3 in einem geringen Abstand voneinander angeordnet sind. Der Abstand zwischen einem aufzunehmenden Objekt und der in den Fig. 3 und 4 eingetragenen Filmebene FE ist somit gegenüber dem Stand der Technik reduziert, so daß Röntgenaufnahmen mit geringerer Unschärfe möglich sind.

Wie aus den Fig. 1 und 2 anhand eines Führungsteiles 10 ersichtlich ist, weisen die Führungsteile 10 jeweils eine T-förmige Führungsschiene 29 auf, wobei die Führungsschienen 29 mit ihrem Steg 30 jeweils zwischen die Blendenplatten 2 und 3 greifen und mit ihren Flanschen 31 die Blendenplatten 2 und 3 quer zu deren Bewegungsrichtung führen. Da außerdem die Blendenplatten 2 und 3 in ihrer Bewegungsrichtung eine Erstreckung aufweisen, die wenigstens ihrer Erstreckung quer zu ihrer Bewegungsrichtung entspricht, ist eine leichtgängige Führung der Blendenplatten 2 und 3 gewährleistet. Senkrecht zu den Ebenen, in denen die Blendenplatten 2 und 3 angeordnet sind, erfolgt deren Führung außer durch die Stege 30 der Führungsschienen 29 durch die Rückwand 6 und die Zwischenwand 7 des Gehäuses 1. Die Zwischenwand 7 besteht übrigens aus einem reibungsarmen Werkstoff und stellt zugleich eine Führung für die Röntgenfilmkassette 5 dar.

Wie aus der Fig. 5 ersichtlich ist, können die Blendenplatten 2 und 3 abweichend von dem zuvor beschriebenen Ausführungsbeispiel mit ihren einander zugewandten Flächen auch unmittelbar aufeinander gleiten. Dabei ist die Blendenplatte 2 an ihrer der Blendenplatte 3 zugewandten Fläche mit einem reibungsarmen Belag 32 versehen, um den Reibungswiderstand, den die Blendenplatten 2 und 3 ihrer Verstellung entgegensetzen, zu vermindern. Zur Führung der Blendenplatten 2 und 3 sind zu deren beiden Seiten in deren Bewegungsrichtung verlaufende U-förmige Führungsschienen 33 an den Führungsteilen 10 vorgesehen, von denen in Fig. 5 nur die eine sichtbar ist und die jeweils beide Blendenplatten 2 und 3 mit ihren parallelen Schenkeln 34 umgreifen und mit ihrem anderen Schenkel 35 quer zu deren Bewegungsrichtung führen.

Die beschriebene Ausbildung der Stelleinrichtung und der Führung für die Blendenplatten 2, 3 ist nur beispielhaft zu verstehen und kann im Rahmen der Erfindung variiert werden. So ist es z.B. nicht unbedingt erforderlich, daß die Blendenplatten 2 und 3 synchron gegeneinander verstellbar sind. Wesentlich für die Erfindung ist, daß die Blendenplatten 2, 3 jeweils eine Blendenöffnung 26 bzw. 27 besitzen und jeweils die Blendenöffnung 26 bzw. 27 der einen Blendenplatte 2 bzw. 3 durch die andere Blendenplatte 3 bzw. 2 zumindest teilweise abdeckbar ist und daß die Blendenöffnung 26 einer der Blendenplatten 2 mit dem Streustrahlenraster 28 versehen ist, da durch diese Maßnahme ein möglichst geringer Abstand zwischen einem aufzunehmenden Objekt und der Filmebene gewährleistet ist.

## Patentansprüche

1. Röntgenzielgerät mit einer Sekundärstrahlenblende, einem wahlweise verwendbaren Streustrahlenraster (28) und einem Kassettenschacht (4), in den eine Röntgenfilmkassette (5) einführbar ist, wobei die Sekundärstrahlenblende zwei Blendenplatten (2, 3) aufweist, die in zueinander parallelen Ebenen angeordnet und mittels einer Stelleinrichtung (12, 13, 14a, 14b, 15a, 15b, 16a, 16b, 21) gegeneinander und übereinander hinweg verstellbar sind, **dadurch gekennzeichnet,** daß die Blendenplatten (2, 3) jeweils eine Blendenöffnung (26, 27) besitzen, wobei die Blendenöffnung (26, 27) jeweils einer Blendenplatte (2, 3) durch die jeweils andere Blendenplatte (3, 2) zumindest teilweise abdeckbar ist, und daß die Blendenöffnung (26) einer der Blendenplatten (2) mit dem Streustrahlenraster (28) versehen ist.

2. Röntgenzielgerät nach Anspruch 1, **dadurch gekennzeichnet,** daß die Stelleinrichtung (12, 13, 14a, 14b, 15a, 15b, 16a, 16b, 21) mit den Blendenplatten (2, 3) in einer gemeinsamen Ebene angeordnet ist.

3. Röntgenzielgerät nach Anspruch 1 oder 2, **dadurch gekennzeichnet,** daß die Blendenplatten (2, 3) mit ihren einander zugewandten Flächen unmittelbar aufeinander gleiten.

4. Röntgenzielgerät nach Anspruch 3, **dadurch gekennzeichnet,** daß eine der Blendenplatten (2) an ihrer der anderen Blendenplatte (3) zugewandten Fläche einen reibungsarmen Belag (32) aufweist.

5. Röntgenzielgerät nach Anspruch 3 oder 4, **dadurch gekennzeichnet,** daß zu beiden Seiten der Blendenplatten (2, 3) in deren Bewegungsrichtung verlaufende U-förmige Führungsschienen (33) vorgesehen sind, die jeweils beide Blendenplatten (2, 3) mit ihren parallelen Schenkeln (34) umgreifen und mit ihrem anderen Schenkel (35) quer zu deren Bewegungsrichtung führen.

6. Röntgenzielgerät nach Anspruch 1 oder 2, **dadurch gekennzeichnet,** daß zu beiden Seiten der Blendenplatten (2, 3) in deren Bewegungsrichtung verlaufende T-förmige Führungsschienen (29) vorgesehen sind, wobei die Führungsschienen (29) jeweils mit ihrem Steg (30) zwischen die Blendenplatten (2, 3) greifen und mit ihren Flanschen (31) die Blendenplatten (2, 3) quer zu deren Bewegungsrichtung führen.

7. Röntgenzielgerät nach Anspruch 5 oder 6, **dadurch gekennzeichnet,** daß die Blendenplatten (2, 3) in ihrer Bewegungsrichtung eine Erstreckung aufweisen, die wenigstens ihrer Abmessung quer dazu entspricht.

8. Röntgenzielgerät nach einem der Ansprüche 1 bis 7, **dadurch gekennzeichnet,** daß die Stelleinrichtung für die Blendenplatten (2, 3) zwei Zugorgane (12, 13) umfaßt, deren jeweilige Enden an verschiedenen Blendenplatten (2, 3) befestigt sind und die derart über Umlenkrollen (14a, 14b, 15a, 15b, 16a, 16b) geführt sind, daß ihre jeweiligen an den Blendenplatten (2, 3) angebrachten Abschnitte (17, 18; 19, 20) parallel zueinander und die an jeweils einer Blendenplatte (2, 3) angebrachten Abschnitte (17, 19; 18, 20) der Zugorgane (12, 13) entgegengesetzt zueinander verlaufen, wobei eines der Zugorgane (13) antreibbar ist.

9. Röntgenzielgerät nach einem der Ansprüche 1 bis 8, **dadurch gekennzeichnet,** daß das Streustrahlenraster (28) streifenförmige Lamellen aufweist, die parallel zur Bewegungsrichtung der Blendenplatten (2, 3) verlaufen.

10. Röntgenzielgerät nach einem der Ansprüche 1 bis 9, **dadurch gekennzeichnet,** daß zwischen der Sekundärstrahlenblende und dem Kassettenschacht (4) eine aus einem reibungsarmen Werkstoff gebildete Zwischenwand (7) zur Führung der Röntgenfilmkassette (5) vorgesehen ist.

## Claims

1. X-ray spotfilm device having a secondary radiation diaphragm, a selectively usable scattered ray grid (28) and a cassette shaft (4) into which an X-ray film cassette (5) can be inserted, in which the secondary radiation diaphragm has two diaphragm plates (2, 3) which are arranged in planes parallel to each other and can be adjusted by means of an adjusting device (12, 13, 14a, 14b, 15a, 15b, 16a, 16b, 21) towards each other and away over each other, characterised in that the diaphragm plates (2, 3) each have a diaphragm opening (26, 27), with it being possible to cover the diaphragm opening (26, 27) of one respective diaphragm plate (2, 3) at least in part by means of the other respective diaphragm plate (3, 2), and in that the diaphragm opening (26) of one of the diaphragm plates (2) is provided with the scattered ray grid (28).

2. X-ray spotfilm device according to claim 1, characterised in that the adjusting device (12, 13, 14a, 14b, 15a, 15b, 16a, 16b, 21) is arranged in a common plane with the diaphragm plates (2, 3).

3. X-ray spotfilm device according to claim 1 or 2, characterised in that the diaphragm plates (2, 3) slide directly one on top of the other with their facing surfaces.

4. X-ray spotfilm device according to claim 3, characterised in that one of the diaphragm plates (2) has a low-friction coating (32) on its surface facing the other diaphragm plate (3).

5. X-ray spotfilm device according to claim 3 or 4, characterised in that provided on both sides of the diaphragm plates (2, 3) there are U-shaped guide rails (33) which extend in the direction of movement of the diaphragm plates (2, 3) and which in each case embrace both diaphragm plates (2, 3) with their parallel legs (34) and with their other leg (35) guide them transversely to their direction of movement.

6. X-ray spotfilm device according to claim 1 or 2, characterised in that provided on both sides of the diaphragm plates (2, 3) there are T-shaped guide rails (29) which extend in the direction of movement of the diaphragm plates (2, 3), with the guide rails (29) engaging in each case with their cross-piece (30) between the diaphragm plates (2, 3) and with their flanges (31) guiding the diaphragm plates (2, 3) transversely to their direction of movement.

7. X-ray spotfilm device according to claim 5 or 6 characterised in that the diaphragm plates (2, 3) have in their direction of movement an extension which corresponds to at least their dimension transversely thereto.

8. X-ray spotfilm device according to one of the claims 1 to 1, characterised in that the adjusting device for the diaphragm plates (2, 3) comprises two traction elements (12, 13), the respective ends of which are secured to different diaphragm plates (2, 3) and which are guided by way of deflection rollers (14a, 14b, 15a, 15b, 16a, 16b) in such a way that their respective sections (17, 18; 19, 20) attached to the diaphragm plates (2, 3) extend parallel to each other and the sections (17, 19; 18, 20) of the traction elements (12, 13) attached to one respective diaphragm plate (2, 3) extend in opposition to each other, with one of the traction elements (13) being drivable.

9. X-ray spotfilm device according to one of the claims 1 to 8, characterised in that the scattered ray grid (28) has strip-like lamellae which extend parallel to the direction of movement of the diaphragm plates (2, 3).

10. X-ray spotfilm device according to one of the claims 1 to 9, characterised in that provided between the secondary radiation diaphragm and the cassette shaft (4) there is an intermediate wall (7), which is formed of a low-friction material, for the purpose of guiding the X-ray film cassette (5).

## Revendications

1. Sélecteur radiologique comportant un diaphragme du rayonnement secondaire, une grille antidiffusion (28) pouvant être utilisée, au choix, et un logement (4) pour cassette, dans lequel peut être insérée une cassette à film radiographique (5), le diaphragme du rayonnement secondaire comportant deux plaques collimatrices (2,3), qui sont disposées dans des plans parallèles et qui peuvent être déplacées l'une contre l'autre et l'une au-dessus de l'autre au moyen d'un dispositif de réglage (12,13,14a,14b,15a,15b,16a,16b, 21), caractérisé par le fait que les plaques collimatrices (2,3) possèdent chacune une ouverture de diaphragme (26,28), qui peut être recouverte au moins partiellement par l'autre plaque collimatrice respective (3,2), et que l'ouverture de diaphragme (26) de l'une des plaques collimatrices (2) est équipée de la grille antidiffusante (28).

2. Sélecteur radiologique suivant la revendication 1, caractérisé par le fait que le dispositif de réglage (12,13,14a,14b,15a,15b,16a,16b,21) est disposé dans un plan commun avec les plaques collimatrices (2,3).

3. Sélecteur radiologique suivant la revendication 1 ou 2, caractérisé par le fait que les plaques collimatrices (2,3) glissent directement l'une sur l'autre par leurs surfaces qui sont situées en vis-à-vis.

4. Sélecteur radiologique suivant la revendication 3, caractérisé par le fait que l'une des plaques collimatrices (2) possède un revêtement à faible coefficient de friction (32), sur sa face tournée vers l'autre plaque collimatrice (3).

5. Sélecteur radiologique suivant la revendication 3 ou 4, caractérisé par le fait que des deux côtés des plaques collimatrices (2,3), il est prévu des rails de guidage en forme de U (33), qui s'étendent dans leur direction de déplacement et enserrent, par leurs branches parallèles (34), respectivement les deux plaques collimatrices (2,3) et, par leur autre branche (35), guident les plaques collimatrices transversalement par rapport à leur direction de déplacement.

6. Sélecteur radiologique suivant la revendication 1 ou 2, caractérisé par le fait que des deux côtés des plaques collimatrices, il est prévu des rails de guidage en forme de T (29), qui s'étendent dans leur direction de déplacement et s'engagent respectivement par leur barre transversale (30) entre les plaques collimatrices (2,3) et, par leurs brides (31), guident les plaques collimatrices (2,3) transversalement par rapport à leur direction de déplacement.

7. Sélecteur radiologique suivant la revendication 5 ou 6, caractérisé par le fait que les plaques collimatrices (2,3) possèdent, dans leur direction de déplacement, une étendue qui correspond au moins à leurs dimensions transversalement par rapport à cette direction.

8. Sélecteur radiologique suivant l'une des revendications 1 à 7, caractérisé par le fait que le dispositif de réglage pour les plaques collimatrices (2,3) comprend deux organes de traction (12,13), dont les extrémités respectives sont fixées sur des plaques collimatrices différentes (2,3) et qui sont guidées par l'intermédiaire de galets de renvoi (14a,14b,15a,15b,16a,16b) de telle sorte que leurs sections respectives (17,18;19,20) montées sur les plaques collimatrices (2,3) sont parallèles entre elles et que les sections (17,18;19,20), qui sont montées sur les plaques collimatrices respectives (2,3), s'étendent en sens opposé des organes de traction (12,13), dont l'un (13) peut être entraîné.

9. Sélecteur radiologique suivant l'une des revendications 1 à 8, caractérisé par le fait que la grille antidiffusante (28) possède des lamelles en forme de bandes, qui sont parallèles à la direction de déplacement des plaques collimatrices (2,3).

10. Sélecteur radiologique suivant l'une des revendications 1 à 9, caractérisé par le fait qu'entre le diaphragme du rayonnement secondaire et le logement à cassette (4) est prévue une paroi intermédiaire (7) qui est formée par un matériau présentant un faible coefficient de frottement et sert à guider la cassette à film radiographique (5).
